# EUROPÄISCHE PATENTANMELDUNG

(11) **EP 1 270 574 A1**
(43) Veröffentlichungstag der Anmeldung: **02.01.2003**
(21) Anmeldenummer: 02013463.1
(22) Anmeldetag: 14.06.2002
(51) Int. Cl.: C07D 473/12, A23L 2/60

(54) **Coffein-Kimplexe mit verbessertem Geschmack, Verfahren zu ihrer Herstellung und ihre Verwendung**

(30) Priorität: 25.06.2001 DE 10130505
(71) Anmelder: Nutrinova Nutrition Specialties & Food Ingredients GmbH, 65929 Frankfurt am Main (DE)
(72) Erfinder: Burgard, Andreas, Dr., 65929 Frankfurt am Main (DE)
(74) Vertreter: Schweitzer, Klaus, Dr.

(57) **Zusammenfassung**

Komplexverbindungen bzw. Addukte aus Coffein und Acesulfam, in denen Coffein und Acesulfam im molaren Verhältnis 1 : 1 oder 1 : 2 vorliegen, weisen einen angenehm süßen Geschmack auf und sind für zahlreiche Anwendungen in coffeinhaltigen Nahrungs-, Genuss- oder Arzneimitteln geeignet. Die Verbindungen können durch einfache Umsetzung aus den gelösten Komponenten hergestellt werden.

## Beschreibung

Die vorliegende Erfindung betrifft Komplexverbindungen bestehend aus einem Süßstoff und Coffein, bei denen der bittere Geschmack des Coffeins maskiert ist, ein Verfahren zur Herstellung solcher Komplexe sowie deren Verwendung.

Coffein, ein Xanthinderivat, ist in zahlreichen Lebensmitteln wie z.B. Kaffee, Tee, Colagetränken, "Energy drinks", Guarana sowie Kakao und den daraus hergestellten Produkten enthalten. Coffein wird ferner Lebensmitteln wie z.B. Joghurt, Eiscreme oder Süßwaren als Zusatzstoff wegen seiner anregenden Wirkung hinzugefügt. Coffein wird vollständig resorbiert und erreicht nach 30 bis 60 Minuten seinen Maximalspiegel im Blut. Coffein gelangt kurz nach der Absorption ins Gehirn und entfaltet dort seine anregende Wirkung, die bei Erwachsenen mehrere Stunden anhalten kann. Coffein fördert außerdem die Glycogenolyse und die Lipolyse, so dass dem Organismus zusätzlich mehr Energie bereitgestellt wird. Beim Ermüdeten werden die Ermüdungserscheinungen aufgehoben und die geistigen Leistungen gesteigert (E. Hogervorst und Mitarbeiter, Int. J. Sports Med. 1999, 20, 354-361).

Coffein besitzt allerdings einen unangenehm bitteren Geschmack, so dass sich seine Verwendung in Nahrungs- oder Genussmitteln, z. B. in einem Kaugummi, oft sehr schwierig gestaltet bzw. hierfür spezielle Formulierungen notwendig sind.

So hatte z B. die US-Regierung 1996 einen Forschungsauftrag ausgeboten, einen Kaugummi mit Coffein zu entwickeln. Hintergrund dieses Forschungsauftrages war, eine Alternative zu Kaffe bzw. Cola-Getränken zu finden, die bei Schlafdefiziten in Ausnahmesituationen, wie z.B. Militäreinsätzen, ohne viel Aufwand zu sich genommen werden kann (Lynne Lamberg, Journal of the American Medical Association,Vol 281, 1999, 885-886).

Seit Juni 1998 bietet die US-Firma Amurol Confections Co. das Produkt Stay Alert an, ein Kaugummi mit der Wirkung einer kleinen Tasse Kaffee pro Streifen dieses Kaugummis. Stay Alert wurde 1998 durch die amerikanische Gesundheitsbehörde FDA zugelassen und wird in verschiedenen Geschmacksrichtungen wie Mokka- oder Pfefferminzgeschmack angeboten. Neben Coffein ist in Stay Alert auch der Süßstoff Acesulfam-K zur Geschmacksverbesserung enthalten. Allerdings ist zur Herstellung eines solchen Kaugummis ein sogenannter Premix der einzelnen Komponenten erforderlich. Solche Mischungen aus verschiedenen Komponenten haben jedoch oftmals die Eigenschaft, dass bei Bewegungen eines solchen Gemisches z. B. beim Transport oder in der Dosieranlage aufgrund der unterschiedlichen Kristalle bzw. Stoffeigenschaften der einzelnen Komponenten Entmischungen auftreten können, was dazu führt, dass z. B. ein Kaugummi relativ viel Coffein und wenig oder keinen Süßstoff enthält und umgekehrt. Es stellt sich hier und in anderen Anwendungen das Problem einer möglichst exakten Dosierung der Komponenten Süßstoff und Coffein. Es wäre deshalb wünschenswert, in einem definierten Verhältnis Coffein und Süßstoff auf einfache Weise dosieren zu können, bei der eine Entmischung nicht mehr möglich ist.

In DE-A 1 242 622, EP-A 0 046 506, WO-A 99/04822 und WO-A 00/12067 werden Verbindungen aus Süßstoff und Arzneimittelwirkstoffen mit verbessertem Geschmack beschrieben, wobei jeweils der Süßstoff und der Wirkstoff im molaren Verhältnis 1:1 vorliegen. Es handelt sich dabei um Säureadditionssalze bzw. um ionische Salzverbindungen, in denen das Süßstoffmolekül als Anion vorliegt. Diese werden durch eine Säure-Base-Reaktion hergestellt, wobei der Süßstoff als Säure mit dem basisch reagierenden Wirkstoff umgesetzt wird.

Aufgabe der vorliegenden Erfindung war es, Coffein in einer Form zur Verfügung zu stellen, die dessen einfache Verwendung und Handhabung bzw. Dosierung bei der Herstellung von z. B. Lebens-, Genuss- oder Arzneimitteln ermöglicht, und bei der der bittere Geschmack des Coffeins maskiert oder unterdrückt wird.

Überraschenderweise wurde nun gefunden, dass Coffein (Formel II) mit Acesulfam-H (Formel I), der zu Acesulfam-K korrespondierenden Säure zu definierten Verbindungen reagieren kann. Acesulfam (6-Methyl-3,4-dihydro-1,2,3-oxathiazin-4-on-2,2-dioxid) ist insbesondere in der Form des Kaliumsalzes (Acesulfam-K) ein im Handel befindlicher Süßstoff. Sowohl Acesulfam-K als auch Acesulfam-H, die zu Acesulfam-K korrespondierende Säure, können nach bekannten Verfahren hergestellt werden (vgl. DE-A 2453 063, Angew. Chemie 85, 965-973 (1973), EP-A 0 155 634). Bei der oben genannten Umsetzung gelingt nicht nur allein die Herstellung von Komplexen allgemein aus Coffein und Acesulfam, sondern es lassen sich ebenso definierte Verbindungen aus einem Molekül Coffein und einem oder zwei Molekülen Acesulfam herstellen. Die angegebenen molaren 1:1- und 1:2-Verhältnisse von Coffein zu Acesulfam innerhalb dieser definierten Komplexe konnten mittels ¹H-NMR bestätigt werden. Diese definierten Verbindungen sind besonders bevorzugt. Ferner umfasst die vorliegende Erfindung auch die entsprechenden Solvate sämtlicher Komplexverbindungen. Überaschenderweise wurde per Röntgenstrukturanalyse festgestellt, dass es sich bei diesen Komplexen aus Coffein und Acesulfam nicht um Salze handelt, was eigentlich aus einer Säure-Base-Reaktion zu erwarten wäre, sondern um nichtionische Coffein-Acesulfam-Addukte, die bevorzugt im molaren Verhältnis 1:1 und 1:2 vorliegen Diese Coffein-Acesulfam-Addukte liegen somit nicht als Salze vor, sondern sind charakterisiert durch Wasserstoff-Brückenbindungen zwischen einem Coffeinmolekül und z. B. einem bzw. zwei Acesulfam-H-Molekülen. Das Acesulfam-H liegt nach Röntgenstrukturanalyse in diesen Addukten in seiner Enolform vor.
Das nachfolgende Reaktionsschema dient zur Veranschaulichung dieses Sachverhalts:

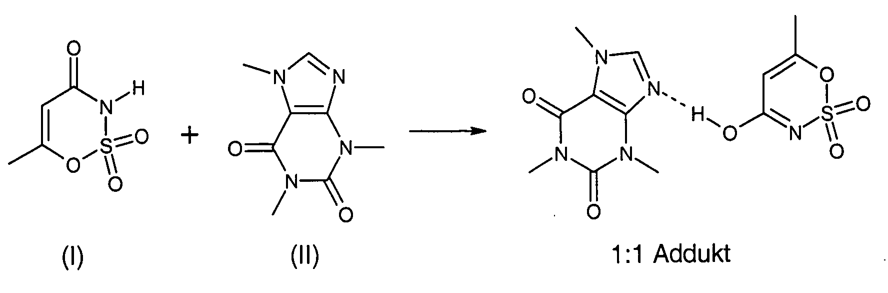

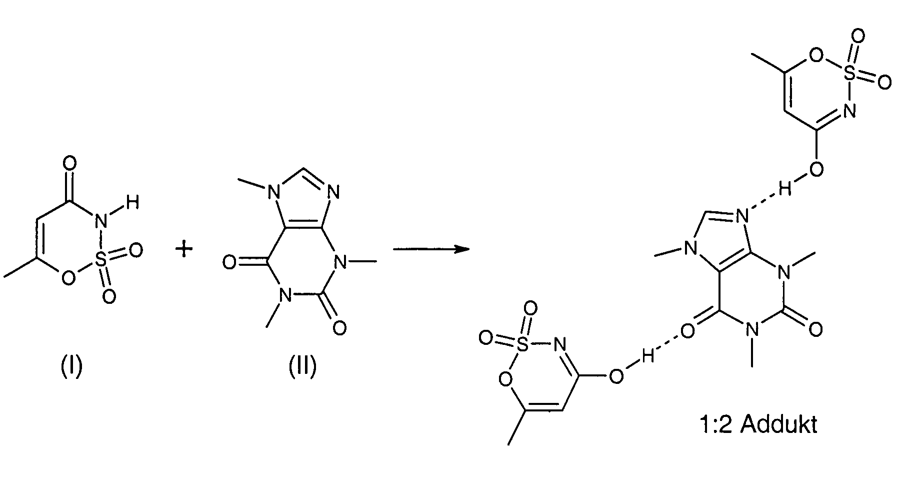

Überraschenderweise zeichnen sich alle diese Verbindungen sowie deren Solvate durch einen angenehmen Süßgeschmack aus, wobei die unangenehme Geschmackskomponente des Coffeins beim 1:1-Addukt im Vergleich zum Coffein ohne Acesulfam deutlich gemindert ist und beim 1:2-Coffein-Acesulfam-Addukt zunächst durch einen zitrusartigen Süßgeschmack vollständig maskiert wird. Der sich anschließende leicht bittere Nachgeschmack des Coffeins ist sehr deutlich vermindert.

Die Adduktbildung aus Coffein und Acesulfam bevorzugt in einem definierten Verhältnis führt nicht nur zu einer Verminderung bzw. Maskierung des unangenehmen bitteren Geschmacks des Coffeins, sondern die definierten 1:1- bzw. 1:2-Coffein-Acesulfam-Addukte können nun z.B. in Nahrungs-, Genuss- oder Arzneimitteln, wie z. B. Kaugummis, ohne die Gefahr einer Entmischung eingearbeitet werden. Das Problem der Entmischung bei Transport oder bei dem Dosiervorgang entfällt ebenfalls.

Damit ist auch sichergestellt, dass die so hergestellten Endprodukte den gewünschten einheitlichen bzw. festgelegten Gehalt an Coffein und Süßstoff enthalten.

Die Verarbeitung der erfindungsgemäßen Addukte in der Nahrungs-, Genuss- oder Arzneimittelindustrie erfordert gegenüber dem Zusatz von Coffein allein oder von Coffein und Süßstoff als getrennt vorliegenden Stoffen keine besonderen Vorkehrungen, sondern erfolgt nach den dort üblichen Verfahren. Dies gilt auch grundsätzlich für Arzneimittelformulierungen, welche diese Stoffe enthalten.

Die vorliegende Erfindung umfasst somit auch fest oder flüssige Zubereitungen, wie z. B. Nahrungs- und Genussmittel z. B. in Form von Kaugummi, Kautabletten oder Komprimaten, oder auch Arzneimittel, welche die erfindungsgemäßen Komplexe bzw. Addukte und/oder deren Solvate enthalten. Ferner werden die entsprechenden Vormischungen oder Premix, welche diese Verbindungen enthalten und bei der Herstellung von Nahrungs-, Genuss oder Arzneimitteln verwendet werden, von der vorliegenden Erfindung umfasst.

Die Darstellung der Coffein-Süßstoff- bzw. Coffein-Acesulfam-Addukte gelingt sehr einfach z. B. aus Lösungen, bevorzugt aus wässrigen Lösungen des Coffeins und des Acesulfam-H, in denen diese Stoffe bevorzugt im molaren Verhältnis 1:1 bzw. 1:2 vorliegen. Die erhaltenen Reaktionslösungen werden in geeigneter Weise, z. B. im Vakuum, vom Lösungsmittel befreit. Es resultieren jeweils die entsprechenden Coffein-Acesulfam-Addukte als farblose Kristalle, die nach ¹H-NMR-Spektroskopie im molaren Verhältnis 1:1- bzw. 1:2-Coffein-Acesulfam vorliegen. Auf diese Weise können auch die entsprechenden Solvate erhalten werden.

Als Lösungsmittel oder Lösungsmittelgemisch werden bevorzugt Wasser und/oder mit Wasser mischbare Lösungsmittel wie z. B. Alkohole verwendet. Die Reaktionstemperaturen liegen bevorzugt bei 20 bis 100 °C, besonders bevorzugt bei 30 bis 70 °C, ganz besonders bevorzugt bei 40 bis 60 °C.

Die nachfolgenden Beispiele sollen die Erfindung näher erläutern.

### Beispiel 1

### Herstellung eines 1:1-Adduktes aus Coffein und Acesulfam-H

In 20 ml Wasser werden 4 mmol (0,785 g) Coffein mit 4 mmol (0,653 g) Acesulfam-H bei 55 °C gelöst. Das Reaktionsgemisch wird anschließend im Vakuum eingeengt. Es resultieren mit 100 % Ausbeute farblose Kristalle, welche laut ¹H-NMR als 1:1-Addukt vorliegen.
60-MHz-¹H-NMR (D₂O): δ(ppm) = 2.2 (s, 3H, CH₃-Acesulfam), 3.35 (s, 3H, CH₃-Coffein), 3.52 (s, 3H, CH₃-Coffein), 4.02 (s, 3H, CH₃-Coffein) 5.95 (s, 1H, CH-Acesulfam), 8.25 (s, 1H, CH-Coffein)

### Beispiel 2

### Herstellung eines 1:2-Adduktes aus Coffein und Acesulfam-H

In 20 ml Wasser werden 4 mmol (0,785 g) Coffein mit 8 mmol (1,306 g) Acesulfam-H bei 55 °C gelöst. Das Reaktionsgemisch wird anschließend im Vakuum eingeengt. Es resultieren mit 100 % Ausbeute farblose Kristalle, welche laut ¹H-NMR als 1:2-Addukt vorliegen.
60-MHz-¹H-NMR (D₂O): δ(ppm) = 2.2 (s, 6H, CH₃-Acesulfam), 3.35 (s, 3H, CH₃-Coffein), 3.52 (s, 3H, CH₃-Coffein), 4.02 (s, 3H, CH₃-Coffein) 5.95 (s, 2H, CH-Acesulfam), 8.25 (s, 1H, CH-Coffein)

## Patentansprüche

1. Verbindung aus Coffein und Acesulfam-H und deren Solvate.

2. Verbindung nach Anspruch 1, **dadurch gekennzeichnet, dass** das molare Verhältnis von Coffein zu Acesulfam-H 1 : 1 oder 1 : 2 beträgt.

3. Verbindung nach Anspruch 1 oder 2, **dadurch gekennzeichnet, dass** es sich um eine nicht-ionische Verbindung handelt.

4. Feste oder flüssige Zubereitung, **dadurch gekennzeichnet, dass** sie eine Verbindung nach einem oder mehreren der Ansprüche 1 bis 3 enthält.

5. Zubereitung nach Anspruch 4, **dadurch gekennzeichnet, dass** es sich um ein Nahrungs-, Genuss- oder Arzneimittel oder einen zu deren Herstellung verwendbaren Premix handelt.

6. Zubereitung nach Anspruch 4 oder 5, **dadurch gekennzeichnet, dass** es sich um einen Kaugummi, eine Kautablette oder ein Komprimat handelt.

7. Verwendung einer Verbindung nach einem oder mehreren der Ansprüche 1 bis 3 zur Herstellung einer Zubereitung nach einem oder mehreren der Ansprüche 4 bis 6.

8. Verfahren zur Herstellung einer Verbindung nach einem oder mehreren der Ansprüche 1 bis 3 durch Umsetzung von Coffein und Acesulfam-H in dem gewünschten molaren Verhältnis in einem geeigneten Lösungsmittel oder Lösungsmittelgemisch und gegebenenfalls anschließender Isolierung des gebildeten Reaktionsproduktes.

9. Verfahren nach Anspruch 8, **dadurch gekennzeichnet, dass** als Lösungsmittel Wasser und/oder mit Wasser mischbare Lösungsmittel verwendet werden.
